# EUROPÄISCHE PATENTSCHRIFT

(11) **EP 3 104 832 B1**
(45) Veröffentlichungstag und Bekanntmachung des Hinweises auf die Patenterteilung: **30.01.2019**
(21) Anmeldenummer: 15702499.3
(22) Anmeldetag: 05.02.2015
(51) Int. Cl.: A61K 8/39, A61Q 17/04, A61K 8/06

(54) **STABILE KOSMETISCHE HYDRODISPERSION**
STABLE COSMETIC HYDRODISPERSION
HYDRODISPERSION COSMETIQUE STABLE

(30) Priorität: 10.02.2014 DE 102014202377
(43) Veröffentlichungstag der Anmeldung: 21.12.2016
(73) Patentinhaber: Beiersdorf AG, 20253 Hamburg (DE)
(72) Erfinder: WRAGE, Tanja, 22337 Hamburg (DE); BORCHERS, Kathrin, 21614 Buxtehude (DE); LERG, Heike, 22303 Hamburg (DE); SCHULZE, Sabrina, 22081 Hamburg (DE)
(86) Internationale Anmeldenummer: PCT/EP2015/052356
(87) Internationale Veröffentlichungsnummer: WO 2015/118038

(56) Entgegenhaltungen:
- WO-A2-2013/120823
- WO-A2-2013/120825
- WO-A2-2013/120829
- WO-A2-2014/012699
- DE-A1-102011 077 037
- US-A1- 2010 310 617

## Beschreibung

Die vorliegende Erfindung betrifft eine kosmetische Hydrodispersion.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Um Emulsionen über einen längeren Zeitraum stabil zu halten und eine Entmischung der Phasen zu verhindern, werden den Emulsionen sogenannte Emulgatoren zugesetzt. Bei Emulgatoren handelt es sich in der Regel um Moleküle mit einem polaren, hydrophilen Strukturelement und einem unpolaren, lipophilen Strukturelement.

Neben diesen "klassischen" Emulsionen kennt der Fachmann auch sogenannte Hydrodispersionen, die aus einer flüssigen Lipidphase in einer äußeren wässrigen Phase bestehen. Bei diesen Hydrodispersionen wird die Stabilität des Mehrphasensystems üblicherweise dadurch gewährleistet, dass in der wässrigen Phase mit Hilfe eines Gelbildners ein Gelgerüst aufgebaut wird, in welchem die Lipidtröpfchen stabil suspendiert sind. Ein typischer Gelbildner hierfür ist beispielsweise das Polymer mit der INCI-Bezeichnung Acrylates/C10-30 Alkyl Acrylates Crosspolymer, welches ein netzförmiges Gerüst in der Dispersion aufbaut.

Nachteilig am Stande der Technik ist der Umstand, dass die mit einem Gelbildner stabilisierten Hydrodispersionen bei starker thermischer und mechanischer Belastung nicht besonders stabil sind, sondern zu Ölabscheidungen neigen. Diese Ölabscheidungen treten besonders häufig bei O/W-Hydrodispersionen (d.h. bei Hydrodispersionen mit äußerer wässriger Phase) auf, die eine große Ölphase aufweisen. Dieser Effekt lässt sich sehr gut im Labor mittels "Lumifugen"-Test in einer Art Zeitraffer simulieren.

Es war daher die Aufgabe der vorliegenden Erfindung, kosmetische Öl-in-Wasser Hydrodispersionen (O/W-Hydrodispersionen) über das bereits bekannte Niveau weiter zu stabilisieren. Insbesondere sollten O/W Hydrodispersionen mit einem hohem Ölphasenanteil, d.h. mit einem Ölphasenanteil von mehr als 25 Gew.-% weiter stabilisiert werden.

Überraschend gelöst wird die Aufgabe durch eine kosmetische Öl-in-Wasser-Hydrodispersion gemäß Anspruch 1.
Zwar kennt der Stand der Technik die WO2014/012699, WO 2013/120829, WO 2013/120823, WO 2013/120825, US 2010/310617 und DE 102011077037, doch konnten diese Schriften nicht den Weg zur vorliegenden Erfindung weisen.

Trotz des Zusatzes eines W/O-Emulgators liegt die erfindungsgemäße Hydrodispersion weiterhin als O/W-System vor.

Überraschend war ferner, dass sich der Lichtschutzfaktor (SPF) der Zubereitung, wenn es sich dabei um eine UV-Filter-haltige Zusammensetzung handelt, signifikant erhöht wird. UV-Filter-haltige Zubereitungen, d.h. Tagespflegeprodukte und Sonnenschutzmittel stellen die erfindungsgemäß bevorzugten Ausführungsformen der vorliegenden Erfindung dar.

Dabei ist eine kosmetische Hydrodispersion, die dadurch gekennzeichnet ist, dass die Zubereitung 0,15 bis 0,5 Gewichts-%, bezogen auf das Gesamtgewicht der Zubereitung Diisostearoyl Polyglyceryl-3 Dimer Dilinoleate enthält, erfindungsgemäß bevorzugt.

Ebenfalls erfindungsgemäß bevorzugt ist es, wenn die erfindungsgemäße Hydrodispersion neben Diisostearoyl Polyglyceryl-3 Dimer Dilinoleate keine weiteren W/O-Emulgatoren und idealerweise überhaupt keine weiteren Emulgatoren enthält.

Das erfindungsgemäße Diisotearoyl Polyglyceryl-3 Dimer Dilinoleate kann beispielsweise unter dem Handelsnamen ISOLAN PDI bei der Firma Evonik erworben werden.

Es ist vorteilhaft im Sinne der vorliegenden Erfindung, wenn die Ölphase der Hydrodispersion mindestens 30 Gewichts-%, bezogen auf das Gesamtgewicht der Zubereitung, beträgt.

Erfindungsgemäß vorteilhafte Ausführungsformen der vorliegenden Erfindung sind dadurch gekennzeichnet, dass die Zubereitung Acrylates/C10-30 Alkyl Acrylates als Gelbildner enthält.

In einem solchen Falle ist es erfindungsgemäß vorteilhaft, wenn die Zubereitung von 0,05 bis 2,0 Gewichts-%, bezogen auf das Gesamtgewicht der Zubereitung, Acrylates/C10-30 Alkyl Acrylates als Gelbildner enthält, wobei eine Einsatzmenge von 0,1 bis 1,0 Gewichts-% bezogen auf das Gesamtgewicht der Zusammensetzung, erfindungsgemäß bevorzugt ist.

Es ist erfindungsgemäß vorteilhaft, wenn die Zubereitung Hydroxyethylcellulose und/oder Polyacrylsäure/Polyacrylat (Carbomer) enthält.

Enthält die Zubereitung Hydroxyethylcellulose, so ist es erfindungsgemäß bevorzugt, wenn die Einsatzkonzentration von 0,01 bis 1,0 Gewichts-%, bezogen auf das Gesamtgewicht der Zubereitung beträgt.

Enthält die Zubereitung Polyacrylsäure/Polyacrylat (Carbomer), so ist es erfindungsgemäß bevorzugt, wenn die Einsatzkonzentration von 0,01 bis 2,0 Gewichts-%, bezogen auf das Gesamtgewicht der Zubereitung beträgt.

Enthält die Zubereitung Hydroxyethylcellulose und Polyacrylsäure/Polyacrylat (Carbomer), so sollte, erfindungsgemäß vorteilhaft, die Gesamtmenge aus beiden Komponenten zwischen 0,1 und 1,5 Gewichts-%, bezogen auf das Gesamtgewicht der Zubereitung, gewählt werden.

Es ist erfindungsgemäß vorteilhaft, wenn die Zubereitung UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 2-Ethylhexyl-2-cyano-3,3-diphenylacrylat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tris-(biphenyl)-1,3,5-triazin; 2,2'-Methylen-bis-(6-(2H-benzotriazol-2-yl)-4-(1,1,3,3-tetramethylbutyl)-phenol); 4-Dicyanomethylen-2,6-dimethyl-1,4-dihydropyridin-N-(ethyloxysulfatestersalze), Titandioxid; Zinkoxid; Polysiloxancopolymer mit einer statistischen Verteilung gemäß der Formel:

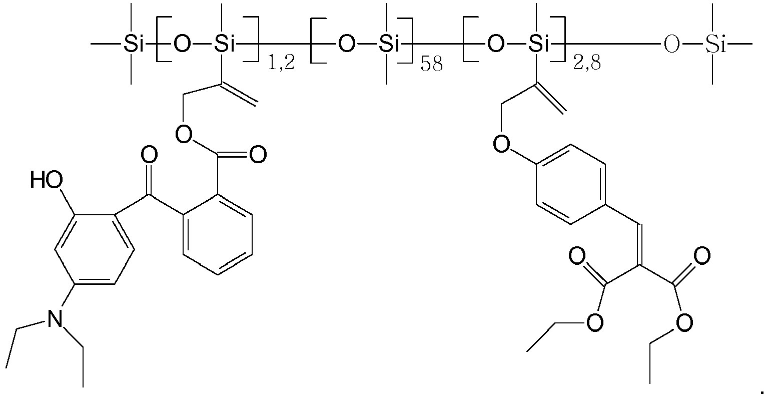

Dabei ist es erfindungsgemäß bevorzugt, wenn die Zubereitung kein 3-(4-Methylbenzyliden)-campher und 2-Hydroxy-4-methoxybenzophenon (INCI: Oxybenzon) enthält, also frei von diesen Inhaltsstoffen ist.

Es ist erfindungsgemäß vorteilhaft, wenn die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Menthol, Glycerylglycose, (2-Hydroxyethyl)harnstoff, Vitamin E bzw. seine Derivate, Hyaluronsäure und/oder deren Salze und/oder Licochalcon A enthält.

Es ist erfindungsgemäß von Vorteil, wenn die erfindungsgemäße Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Erfindungsgemäß vorteilhafte Ausführungsformen der vorliegenden Erfindung sind ferner dadurch gekennzeichnet, dass die Zubereitung Ethanol, Phenoxyethanol und/oder Ethylhexylglycerin enthält.

Enthält die erfindungsgemäße Zubereitung Ethanol, so ist es vorteilhaft im Sinne der vorliegenden Erfindung, wenn die Einsatzmenge von 5,0 bis 15,0 Gewichts-%, bezogen auf das Gesamtgewicht der Zubereitung, beträgt.

Es ist erfindungsgemäß vorteilhaft, wenn die erfindungsgemäße Zubereitung Silica Dimethyl Silylate und/oder Tapiokastärke enthält.

Enthält die Zubereitung Silica Dimethyl Silylate, so ist es erfindungsgemäß bevorzugt, wenn die Einsatzkonzentration von 0,5 bis 3,0 Gewichts-%, bezogen auf das Gesamtgewicht der Zubereitung beträgt.

Enthält die Zubereitung Tapiokastärke, so ist es erfindungsgemäß bevorzugt, wenn die Einsatzkonzentration von 2 bis 6,0 Gewichts-%, bezogen auf das Gesamtgewicht der Zubereitung beträgt.

Es ist erfindungsgemäß von Vorteil, wenn die erfindungsgemäße Zubereitung frei ist von Polyethylenglycol, Polyethylenglycolethern und Polyethylenglycolestern (sogenannten PEG-Derivaten).

Erfindungsgemäß vorteilhafte Ausführungsformen der vorliegenden Erfindung sind dadurch gekennzeichnet, dass die die Zubereitung frei ist von Parabenen, Methylisothiazolinon, Chlormethylisothiazolinon und DMDM-Hydantoin.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäße Ölphase kann beispielsweise ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthalten.

Erfindungsgemäß ist die Verwendung von Diisostearoyl Polyglyceryl-3 Dimer Dilinoleate in einer kosmetischen Zubereitung nach einem der vorhergehenden Ansprüche zur Erhöhung der Stabilität und Verhinderung der Ölabscheidung.

Erfindungsgemäß ist außerdem Verwendung von Diisostearoyl Polyglyceryl-3 Dimer Dilinoleate in einer kosmetischen Zubereitung nach einem der vorhergehenden Ansprüche zur Erhöhung des Lichtschutzfaktors (SPF), wenn es sich bei der erfindungsgemäßen Zubereitung um eine UV-Filter-haltige Zusammensetzung handelt.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **1** | **2** | **3** | **4** | **5** |
|---|---|---|---|---|---|
| Diisostearoyl Polyglyceryl-3 Dimer Dilinoleate | 0,10 | 0,10 | 0,3 | 0,5 | 1 |
| Homosalate | 9,50 | 9,50 | 9,50 | 9,50 | 9,50 |
| Octocrylene | 7,50 | 9,5 | 7,50 | 7,50 | 7,50 |
| Ethylhexyl Salicylate | 4,75 | 3,0 | 4,75 | 4,75 | 4,75 |
| Bis-Ethylhexyloxyphenol Methoxyphenyl Triazine | 2,00 | 2,00 | 1,5 | 2,00 | 0,75 |
| Polysilicone-15 | 1,00 | 0,8 | 1,00 | 0,5 | 1,00 |
| Butyl Methoxydibenzoylmethane | 4,50 | 3 | 3,5 | 4 | 4,50 |
| Titanium Dioxide (nano) + Trimethoxycaprylylsilane | 1 | 2,00 | 2,00 | 4 | 2 |
| Tocopheryl Acetate | 1 | 0,5 | 1 | 0,11 | 0,11 |
| Isopropyl Palmitate | 4 | 5 | 3,00 | 2,50 | 2,50 |
| Silica Dimethyl Silylate | 1,00 | 2 | 1,00 | 1,50 | 1,00 |
| Tapioca Starch + Aqua | 4,00 | 2 | 2 | 4,00 | 3 |
| Parfum | 0,40 | 0,40 | 0,40 | 0,7 | 0,6 |
| Glycerin + Aqua | 1,00 | 1,00 | 10 | 1,00 | 1,00 |
| Aqua + Sodium Hydroxide | 0,27 | 0,27 | 0,27 | 0,27 | 0,27 |
| Methylparaben | 0,40 | 0,40 | 0,40 | 0,00 | 0,00 |
| Phenoxyethanol | 0,00 | 0,00 | 0,00 | 0,50 | 0,50 |
| Hydroxyethylcellulose | 0,10 | 0,10 | 0,50 | 0,10 | 0,30 |
| Carbomer | 0,3 | 0,10 | 0,6 | 0,10 | 0,05 |
| Acrylates/C10-30 Alkyl Acrylate Crosspolymer | 0,20 | 0,20 | 0,20 | 0,20 | 0,20 |
| Aqua ad | 100 | 100 | 100 | 100 | 100 |
| Alcohol Denat. + Aqua | 10,00 | 8 | 9 | 7 | 10,00 |
| Trisodium EDTA | 1,00 | 1,00 | 1,00 | 1,00 | 1,00 |

### Lumifugentest

Für die Stabilitätsanalyse kommt eine optische temperierbare "Lumifuge" (Fa. LUMGmbH) zum Einsatz. Verwendet werden Standardeinwegmesszellen aus Polycarbonat (2x8mm), die mit 4 mL Probenvolumen gefüllt sind. Die Messungen findet bei 40°C und Lichtfaktor 1 statt. Die Proben werden zwischen 12 und 24 h bei einer Drehzahl von 2082 rpm gemessen. Ausgewertet werden die optischen Eigenschaften der Proben in den PC-Küvetten nach der zentrifugalen Beanspruchung.

Ohne Emulgator entstehen in der Küvette 3 Phasen. Beginnend vom Küvettenboden mit einer undurchsichtigen Phase, gefolgt von einer klaren Phase, die an der Oberfläche abgeschlossen wird von einer transluzenten Phase.
Mit Emulgator ist keine Auftrennung in Phasen erkennbar. Es existiert nur eine undurchsichtige Phase.

## Patentansprüche

1. Kosmetische Öl-in-Wasser-Hydrodispersion enthaltend einen Wasser-in-Öl-Emulgator in einer Konzentration von 0,01 bis 1 Gewichts-%, bezogen auf das Gesamtgewicht der Zubereitung, wobei als Wasser-in-ÖI-Emulgator Diisostearoyl Polyglyceryl-3 Dimer Dilinoleate eingesetzt wird.

2. Kosmetische Hydrodispersion nach Anspruch 1, **dadurch gekennzeichnet, dass** die Zubereitung 0,15 bis 0,5 Gewichts-%, bezogen auf das Gesamtgewicht der Zubereitung Diisostearoyl Polyglyceryl-3 Dimer Dilinoleate enthält.

3. Kosmetische Hydrodispersion nach einem der vorhergehenden Ansprüche, **dadurch gekennzeichnet, dass** die Ölphase der Hydrodispersion mindestens 30 Gewichts-%, bezogen auf das Gesamtgewicht der Zubereitung, beträgt.

4. Kosmetische Hydrodispersion nach einem der vorhergehenden Ansprüche, **dadurch gekennzeichnet, dass** die Zubereitung Acrylates/C10-30 Alkyl Acrylates als Gelbildner enthält.

5. Kosmetische Hydrodispersion nach einem der vorhergehenden Ansprüche, **dadurch gekennzeichnet, dass** die Zubereitung von 0,05 bis 2 Gewichts-% bezogen auf das Gesamtgewicht der Zubereitung, Acrylates/C10-30 Alkyl Acrylates als Gelbildner enthält.

6. Kosmetische Hydrodispersion nach einem der vorhergehenden Ansprüche, **dadurch gekennzeichnet, dass** die Zubereitung Hydroxyethylcellulose und/oder Polyacrylsäure/Polyacrylat (Carbomer) enthält.

7. Kosmetische Zubereitung nach einem der vorhergehenden Ansprüche, **dadurch gekennzeichnet, dass** die Zubereitung UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxyldisiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 2-Ethylhexyl-2-cyano-3,3-diphenylacrylat; 3-(4-(2,2-bis Ethoxycarbonylvinyl)-phenoxy)propenyl)-methoxysiloxan/ Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoäsäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-([4-(2-ethyl-hexyloxy)-2-hydroxy]-phenyl)-6-(4-methoxyphenyl)-1,3,5-triazin, 2,4,6-Tris-(biphenyl)-1,3,5-triazin; 2,2'-Methylen-bis-(6-(2H-benzotriazol-2-yl)-4-(1,1,3,3-tetramethylbutyl)-phenol); 4-Dicyanomethylen-2,6-dimethyl-1,4-dihydropyridin-N-(ethyloxysulfatestersalze), Titandioxid; Zinkoxid; Polysiloxancopolymer mit einer statistischen Verteilung gemäß der Formel:

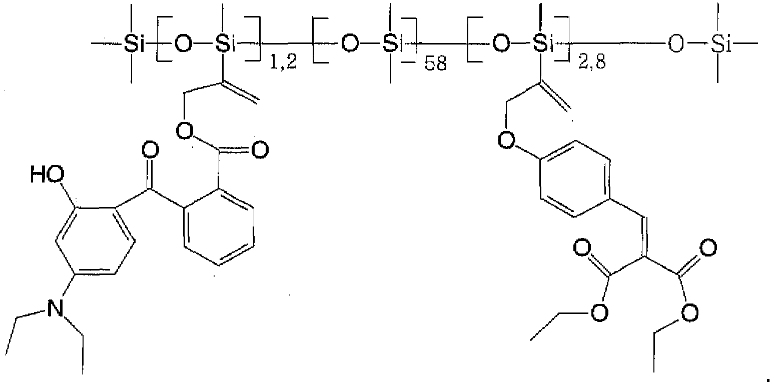

8. Kosmetische Zubereitung nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Menthol, Glycerylglycose, (2-Hydroxyethyl)harnstoff, Vitamin E bzw. seine Derivate, Hyaluronsäure und/oder deren Salze und/oder Licochalcon A enthält.

9. Kosmetische Zubereitung nach einem der vorhergehenden Ansprüche, **dadurch gekennzeichnet, dass** die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

10. Kosmetische Zubereitung nach einem der vorhergehenden Ansprüche, **dadurch gekennzeichnet, dass** die Zubereitung frei ist von Polyethylenglycol, Polyethylenglycolethern und Polyethylenglycolestern (sogenannten PEG-Derivaten).

11. Kosmetische Zubereitung nach einem der vorhergehenden Ansprüche, **dadurch gekennzeichnet, dass** die Zubereitung Ethanol, Phenoxyethanol und/oder Ethylhexylglycerin enthält.

12. Kosmetische Zubereitung nach einem der vorhergehenden Ansprüche, **dadurch gekennzeichnet, dass** die Zubereitung Silica Dimethyl Silylate und/oder Tapiokastärke enthält.

13. Verwendung von Diisostearoyl Polyglyceryl-3 Dimer Dilinoleate in einer kosmetischen Zubereitung nach einem der vorhergehenden Ansprüche zur Erhöhung der Stabilität und Verhinderung der Ölabscheidung.

14. Verwendung von Diisostearoyl Polyglyceryl-3 Dimer Dilinoleate in einer kosmetischen Zubereitung nach einem der vorhergehenden Ansprüche zur Erhöhung des Lichtschutzfaktors (SPF).

## Claims

1. Cosmetic oil-in-water hydrodispersion comprising a water-in-oil emulsifier at a concentration of 0.01 to 1% by weight, based on the total weight of the preparation, wherein diisostearoyl polyglyceryl-3 dimer dilinoleate is used as water-in-oil emulsifier.

2. Cosmetic hydrodispersion according to Claim 1, **characterized in that** the preparation comprises 0.15 to 0.5% by weight diisostearoyl polyglyceryl-3 dimer dilinoleate, based on the total weight of the preparation.

3. Cosmetic hydrodispersion according to either of the preceding claims, **characterized in that** the oil phase of the hydrodispersion accounts for at least 30% by weight, based on the total weight of the preparation.

4. Cosmetic hydrodispersion according to any of the preceding claims, **characterized in that** the preparation comprises acrylates/C10-30 alkyl acrylates as gel former.

5. Cosmetic hydrodispersion according to any of the preceding claims, **characterized in that** the preparation comprises from 0.05 to 2% by weight, based on the total weight of the preparation, acrylates/C10-30 alkyl acrylates as gel former.

6. Cosmetic hydrodispersion according to any of the preceding claims, **characterized in that** the preparation comprises hydroxyethylcellulose and/or polyacrylic acid/polyacrylate (carbomer).

7. Cosmetic preparation according to any of the preceding claims, **characterized in that** the preparation comprises UV filters selected from the group of compounds comprising phenylene-1,4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydoxybenzoyl)benzoate; 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethylbenzalmalonate; 2-ethylhexyl 2-cyano-3,3-diphenylacrylate; 3-(4-(2,2-bis ethoxycarbonylvinyl)phenoxy)propenyl)methoxysiloxane / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis-{[4-(2-ethylhexyloxy)-2-hydroxy]phenyl}-6-(4-methoxyphenyl)-1,3,5-triazine; 2,4,6-tris(biphenyl)-1,3,5-triazine; 2,2'-methylenebis(6-(2H-benzotriazol-2-yl)-4-(1,1,3,3-tetramethylbutyl)phenol; 4-dicyanomethylene-2,6-dimethyl-1,4-dihydropyridine-N-(ethyloxysulfate ester salts); titanium dioxide; zinc oxide; polysiloxane copolymer having a statistical distribution according to the formula:

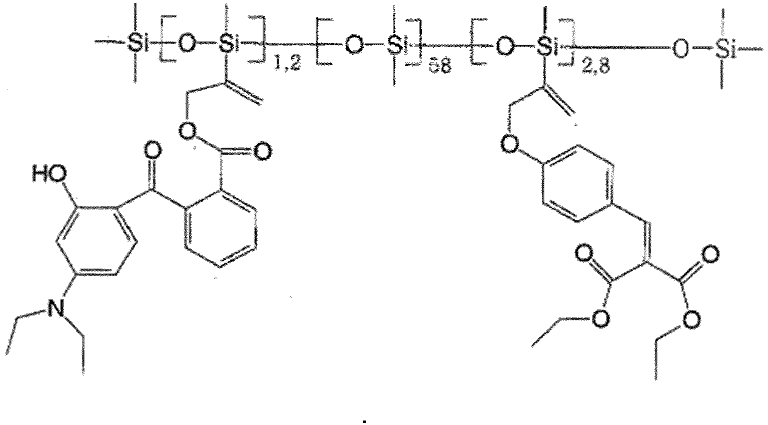

8. Cosmetic preparation according to any of the preceding claims, **characterized in that** the preparation comprises one or more compounds selected from the group of compounds comprising alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, tocopheryl acetate, dihydroxyacetone; 8-hexadecene-1,16-dicarboxylic acid, menthol, glycerylglucose, (2-hydroxyethyl)urea, vitamin E or derivatives thereof, hyaluronic acid and/or salts thereof and/or licochalcone A.

9. Cosmetic preparation according to any of the preceding claims, **characterized in that** the preparation comprises one or more alkanediols from the group of compounds comprising 1,2-pentanediol, 1,2-hexanediol, 1,2-octanediol, 1,2-decanediol, 2-methyl-1,3-propanediol.

10. Cosmetic preparation according to any of the preceding claims, **characterized in that** the preparation is free of polyethylene glycol, polyethylene glycol ethers and polyethylene glycol esters (so-called PEG derivatives).

11. Cosmetic preparation according to any of the preceding claims, **characterized in that** the preparation comprises ethanol, phenoxyethanol and/or ethylhexylglycerin.

12. Cosmetic preparation according to any of the preceding claims, **characterized in that** the preparation comprises silica dimethyl silylate and/or tapioca starch.

13. Use of diisostearoyl polyglyceryl-3 dimer dilinoleate in a cosmetic preparation according to any of the preceding claims for increasing the stability and preventing oil separation.

14. Use of diisostearoyl polyglyceryl-3 dimer dilinoleate in a cosmetic preparation according to any of the preceding claims for increasing the sun protection factor (SPF).

## Revendications

1. Hydrodispersion huile dans eau cosmétique contenant un émulsifiant eau dans huile en une concentration de 0,01 à 1 % en poids, par rapport au poids total de la préparation, le diisostéaroyl-polyglycéryl-3 dimère dilinoléate étant utilisé en tant qu'émulsifiant eau dans huile.

2. Hydrodispersion cosmétique selon la revendication 1, **caractérisée en ce que** la préparation contient 0,15 à 0,5 % en poids, par rapport au poids total de la préparation, de diisostéaroyl-polyglycéryl-3 dimère dilinoléate.

3. Hydrodispersion cosmétique selon l'une quelconque des revendications précédentes, **caractérisée en ce que** la phase huileuse de l'hydrodispersion est d'au moins 30 % en poids, par rapport au poids total de la préparation.

4. Hydrodispersion cosmétique selon l'une quelconque des revendications précédentes, **caractérisée en ce que** la préparation contient acrylates/C10-30 alkyl acrylates en tant que gélifiant.

5. Hydrodispersion cosmétique selon l'une quelconque des revendications précédentes, **caractérisée en ce que** la préparation contient 0,05 à 2 % en poids, par rapport au poids total de la préparation, d'acrylates/C10-30 alkyl acrylates en tant que gélifiant.

6. Hydrodispersion cosmétique selon l'une quelconque des revendications précédentes, **caractérisée en ce que** la préparation contient de l'hydroxyéthylcellulose et/ou de l'acide polyacrylique/polyacrylate (carbomère).

7. Hydrodispersion cosmétique selon l'une quelconque des revendications précédentes, **caractérisée en ce que** la préparation contient des filtres UV, choisis dans le groupe constitué par les composés sels de l'acide phénylène-1,4-bis-(2-benzimidazyl)-3,3'-5,5'-tétrasulfonique ; sels de l'acide 2-phénylbenzimidazol-5-sulfonique ; 1,4-di(2-oxo-10-sulfo-3-bornylidène-méthyl)-benzène et ses sels ; sels de l'acide 4-(2-oxo-3-bornylidène-méthyl)benzène-sulfonique ; sels de l'acide 2-méthyl-5-(2-oxo-3-bornylidène-méthyl)sulfonique ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; salicylate d'éthylhexyle ; acide téréphtalidène-dicamphre-sulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone ; 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque, 4-(tert-butyl)-4'-méthoxydibenzoylméthane ; salicylate d'homomenthyle ; 2-hydroxybenzoate de 2-éthylhexyle ; acrylate de 2-éthylhexyl-2-cyano-3,3-diphényle ; benzalmalonate de diméthicodiéthyle ; acrylate de 2-éthylhexyl-2-cyano-3,3-diphényle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine de n° CAS 288254-16-0) ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI: Ethylhexyl Triazone) ; 2,4-bis-{[4-(2-éthyl-hexyloxy)-2-hydroxy]-phényl}-6-(4-méthoxyphényl)-1,3,5-triazine ; 2,4,6-tris-(biphényl)-1,3,5-triazine, 2,2'-méthylène-bis-(6-(2H-benzotriazol-2-yl)-4-(1,1,3,3-tétraméthylbutyl)-phényl) ; sels de l'ester de N-(éthyloxysulfate) de 4-dicyanométhylène-2,6-diméthyl-1,4-dihydropyridine, dioxyde de titane, oxyde de zinc ; copolymère de polysiloxane ayant une distribution statistique selon la formule :

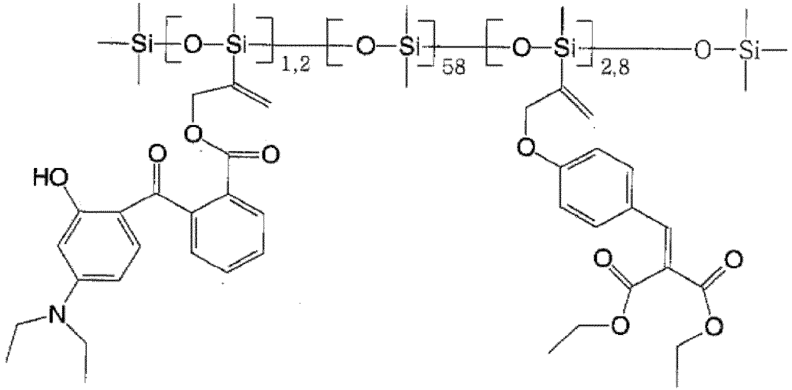

8. Préparation cosmétique selon l'une quelconque des revendications précédentes, **caractérisée en ce que** la préparation contient un ou plusieurs composés choisis dans le groupe constitué par les composés acide alpha-liponique, acide folique, phytoène, D-biotine, coenzyme Q10, alpha-glucosylrutine, carnitine, carnosine, isoflavonoïdes naturels et/ou synthétiques, flavonoïdes créatine, créatinine, taurine, β-alanine, acétate de tocophéryle, dihydroxyacétone, acide 8-hexadécène-1,16-dicarboxylique, menthol, glycérylglucose, (2-hydroxyéthyl)urée, vitamine E ou ses dérivés, acide hyaluronique et/ou ses sels et/ou licochalcone A.

9. Préparation cosmétique selon l'une quelconque des revendications précédentes, **caractérisée en ce que** la préparation contient un ou plusieurs alcane-diols du groupe constitué par les composés 1,2-pentanediol, 1,2-hexanediol, 1,2-octanediol, 1,2-décanediol, 2-méthyl-1,3-propanediol.

10. Préparation cosmétique selon l'une quelconque des revendications précédentes, **caractérisée en ce que** la préparation est exempte de polyéthylène glycol, d'éthers de polyéthylène glycols et d'esters de polyéthylène glycols (dits dérivés de PEG).

11. Préparation cosmétique selon l'une quelconque des revendications précédentes, **caractérisée en ce que** la préparation contient de l'éthanol, du phénoxyéthanol et/ou de l'éthylhexylglycérine.

12. Préparation cosmétique selon l'une quelconque des revendications précédentes, **caractérisée en ce que** la préparation contient du silica diméthyl silylate et/ou de l'amidon de tapioca.

13. Utilisation de diisostéaroyl-polyglycéryl-3 dimère dilinoléate dans une préparation cosmétique selon l'une quelconque des revendications précédentes pour augmenter la stabilité et inhiber la séparation de l'huile.

14. Utilisation de diisostéaroyl-polyglycéryl-3 dimère dilinoléate dans une préparation cosmétique selon l'une quelconque des revendications précédentes pour augmenter le facteur de protection solaire (FPS).
